# EUROPEAN PATENT SPECIFICATION

(11) **EP 3 442 622 B1**
(45) Date of publication and mention of the grant of the patent: **08.04.2020**
(21) Application number: 17725351.5
(22) Date of filing: 11.04.2017
(51) Int. Cl.: A61M 5/20, A61M 5/28, A61M 5/31, A61M 5/315

(54) **INJECTION DEVICE WITH DOSING RESERVOIR FOR A FRACTION OF SOLUTION**
INJEKTIONSVORRICHTUNG MIT DOSIERRESERVOIR FÜR EINEN LÖSUNGSANTEIL
DISPOSITIF D'INJECTION AVEC RÉSERVOIR DE DOSAGE POUR UNE FRACTION DE SOLUTION

(30) Priority: 13.04.2016 IT UA20162549
(43) Date of publication of application: 20.02.2019
(73) Proprietor: Orofino Pharmaceuticals Group S.r.l., 00193 Roma (IT)
(72) Inventor: OROFINO, Ernesto, 00193 Roma (IT)
(74) Representative: Carangelo, Pierluigi
(86) International application number: PCT/IB2017/052073
(87) International publication number: WO 2017/178962

(56) References cited:
- EP-A1- 0 278 015
- WO-A1-2016/012878
- US-A- 3 978 846
- US-A- 4 275 730

## Description

This invention relates to a device for injecting very precise and reduced doses of an injectable solution. Hence the invention relates to the technical field of syringes for injections or of cartridges, of the pre-filled type too, with multiple chambers too for injecting a solution which is reconstituted just before being administered.

Patent application number IT2014RM00408, always by this proprietor, represents an example of known syringes of the pre-filled double-chamber type. Such a syringe allows to inject very reduced doses of a reconstituted solution. Such a known syringe includes one vent outlet for ejecting a fraction of solution, such ejection being necessary to enable to reach an end-administration configuration. Such fraction of solution is ejected alongside the injection of the fraction of administration. During the injection step, the physician is thus forced to close or tampon the vent outlet by means of absorbing material to collect the fraction of solution ejected from the syringe. Such operation has some drawbacks: it makes the injection less simple and it forces the physician to come into contact with the pharmaceutical solution.

The need perceived by the sector of the injection devices provided with a vent outlet is to have available devices able to prevent the dispersion of the ejected fraction during the injection of the administration fraction. For example, WO 2016/012878 A1 discloses a pre-filled injection device for injecting very small doses of a reconstituted solution in an accurate and safe way.

The object of this invention is to overcome the problems of the prior art taking into account the needs of the field.

Such an object is obtained by an injection device according to this invention, provided with a storing cannula for containing the ejected fraction.

The solution according to this invention is particularly advantageous as the storing cannula for containing the ejected fraction can be easily applied to the injection device with no need of substantial changes.

The solution according to this invention is generally suitable for injection devices with two front outlet ducts, and it is particularly suitable for injection devices for very precise and reduced doses of an injectable solution (namely syringes for micro-doses for example doses of one millilitre or of one tenth of millilitre).

Such an object is obtained by an injection device according to claim 1. Dependent claims disclose preferred or advantageous embodiments of the device. The features and the advantages of the injection device according to this invention are made clear from the hereinafter related exemplary but non-limiting disclosure according to the enclosed figures, wherein:
- Figures 1 and 2 show respectively an axonometric projection view and a cross section view of an injection device according to this invention, in one embodiment;
- Figures 3 and 4 show a cross section view of the device of Figure 1 respectively in an initial configuration and in a pre-administering configuration;
- Figures 5 and 6 show respectively an axonometric projection view and a cross section view of an injection device provided with one accessory according to this invention;
- Figures 7 and 8 show a cross section view of the device of Figure 5 respectively in a pre-administering configuration and in an end-administering configuration;
- Figures 9 and 10 show a cross section view of one detail of the device of Figure 1 and of the device of Figure 4 respectively;
- Figure 11 shows one detail of an accessory according to this invention;
- Figure 12 shows one detail of the device of Figure 5;
- Figures 13 and 14 show respectively an axonometric projection view and a cross section view of an injection device according to this invention, in one further embodiment;
- Figures 15 and 16 show a cross section view of the device of Figure 13 respectively in an initial configuration and in a reconstitution configuration of the injectable solution.

In the enclosed figures same or similar elements will be indicated by same reference numbers.

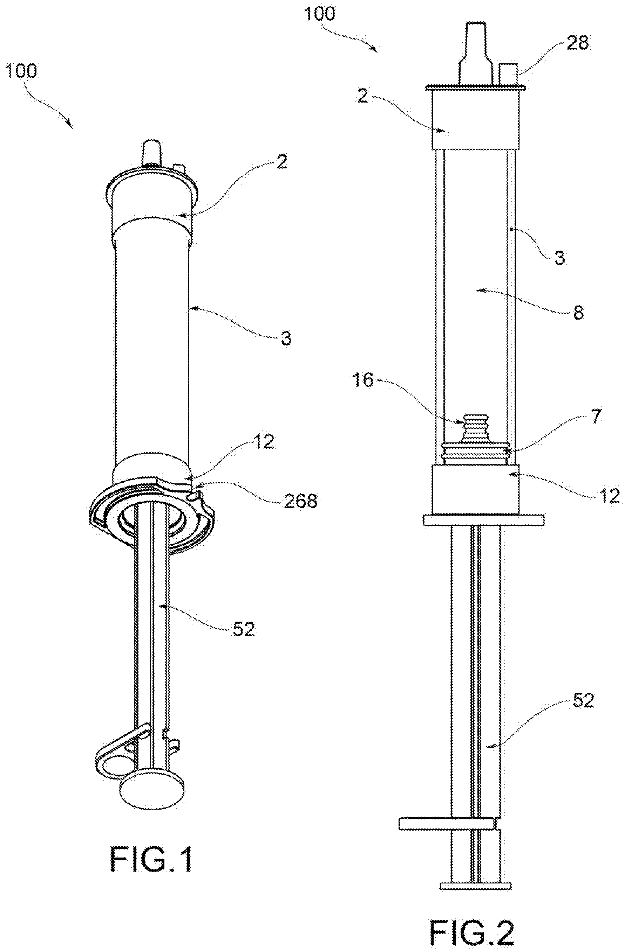

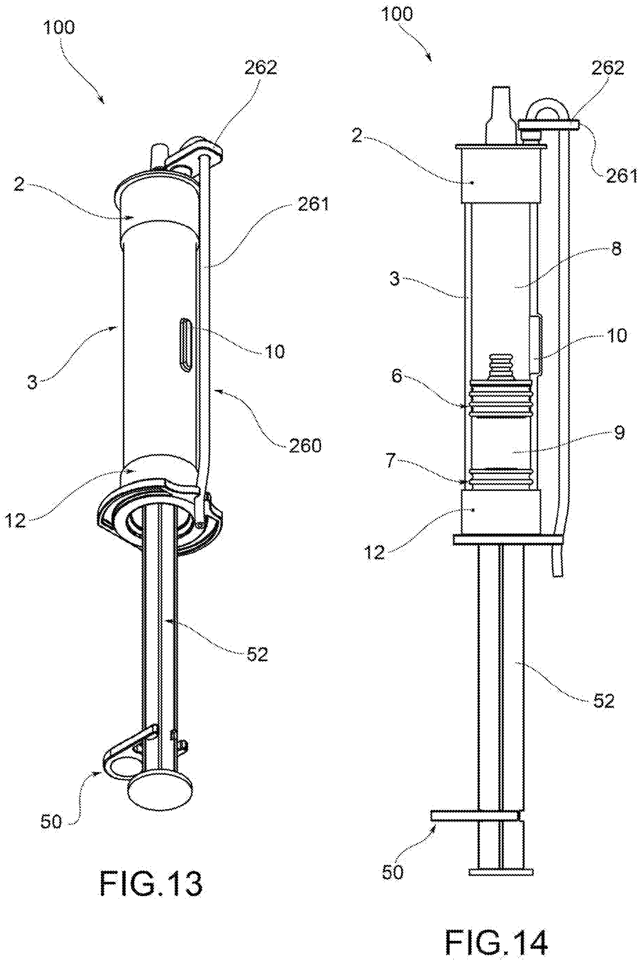

Referring to the enclosed Figures, and in particular to Figures 1 and 13 an injection device is indicated by reference number 100.

In Figures 1 to 4 one embodiment of an injection device 100 is represented, which in the specific represented example, is embodied by a single-chamber syringe. Preferably, the injection device 100 is of the pre-filled type with an injectable solution S. In one alternative embodiment, the injection device 100 is a pre-filled cartridge.

In Figures 13 to 16 a further embodiment of an injection device 100 is represented which in the specific represented example is embodied in a double-chamber pre-filled cartridge. In one alternative embodiment, the injection device is a pre-filled cartridge. The double-chamber injection device is pre-filled with two substances, contained in two originally separated containment chambers, intended to be mixed with each other immediately before being administered to the patient. In order to mix the two substances, the two containment chambers are put in communication with each other in such a way as to enable the reconstitution of the injectable solution. In further embodiments more than two chambers may be provided in the pre-filled injection device.

Preferably, the injection device 100 according to this invention (both in the single-chamber variant and in the double-chamber variant) is suitable for the administration of a very reduced dose of an injectable solution, for example for injecting a quantity of solution smaller than 1 millilitre and preferably of, or almost of, 0.1 millilitre. Preferably the aforesaid dose is smaller than about 1/25 of the injectable solution contained inside the injection device, and preferably of 1/50. For instance, such doses are required to administer antibiotics locally, for example for injecting a cephalosporin antibiotic, such as cefuroxime, into the ocular room of a patient.

Referring to Figure 1, the injection device 100 includes a containment tubular body 3 closed at the front by a closing element 2 and, at the opposite side or at the back, by a grasping portion 12.

The closing element 2 and the grasping portion 12 are fixed to the tubular body 3 or are integrally made with the tubular body 3.

The tubular body 3 is the containment body of a syringe or of a cartridge, it is suitable for containing injectable substances, and it is preferably made of glass or of a transparent or substantially transparent plastic material. Preferably, the tubular body 3 is made in a single piece.

The injection device 100 includes at least one containment chamber 8 suitable for containing an injectable solution S. In the embodiment variant wherein the injection device 100 is of the pre-filled type, the containment chamber 8 contains an injectable solution S. The injection device 100 includes at least one plug 7 arranged within the tubular body 3, so as to delimit the containment chamber 8.

The plug 7 is for example made of plastic material and is such as to sealingly engage with the inner wall of the tubular body 3.

Plug 7 is suitable to slide inside the tubular body 3 under the action of an external pushing or pulling force. Plug 7 is fixed to a plunger 52 so as to slide inside the tubular body 3 under the action of an external pushing or pulling force.

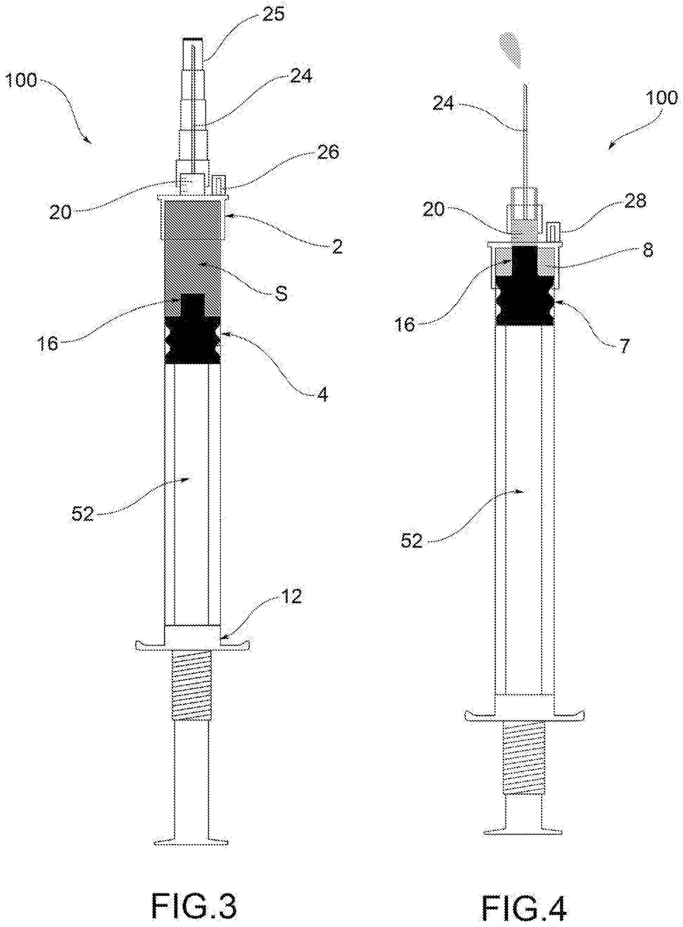

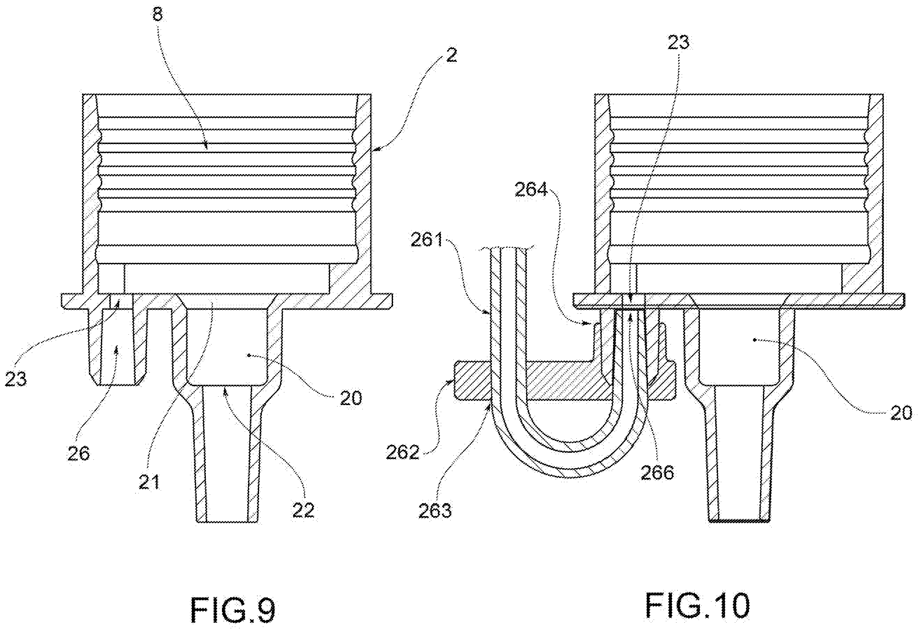

As shown in Figures 3 and 9, the injection device 100 includes a dosing reservoir 20, having one inlet opening 21 and one outlet opening 22. The inlet opening 21 is in communication with the containment chamber 8. In the specific represented example the dosing reservoir 20 is defined within the closing device 2, however in a embodiment variant it may be part of the tubular body 3 itself and it represents a narrowing of the first containment chamber 8.

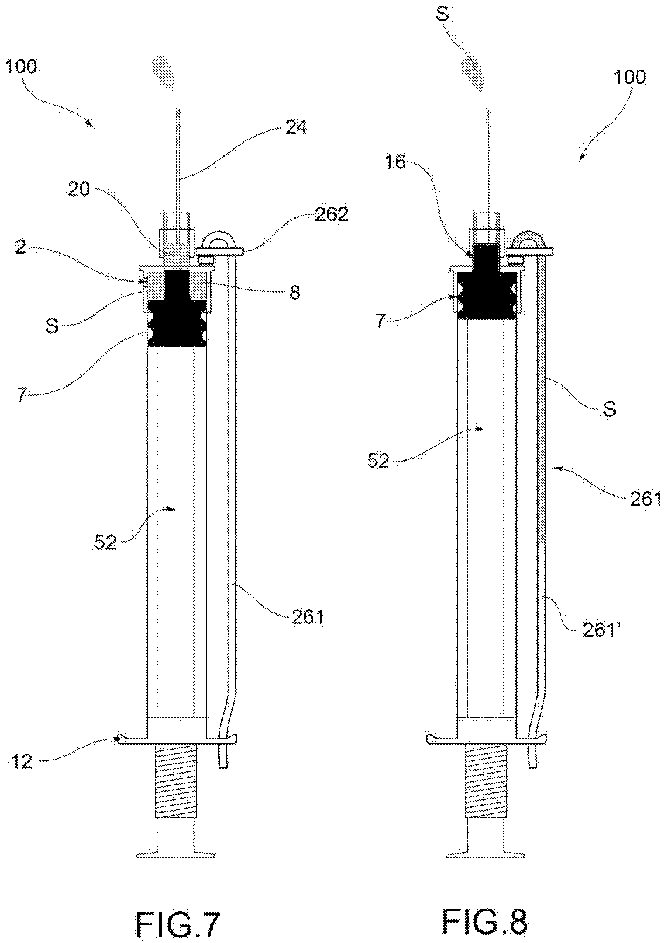

Preferably, the dosing reservoir 20 is made of transparent or translucent material so that its content can be seen from outside, and even more preferably, it includes a graduated scale visible from outside too. Preferably, the injection device 100 includes a needle 24 whose internal channel is in communication with fluid with the outlet opening 22 of the dosing reservoir 20. Preferably, the injection device 100 includes a protective screen 25, or cap, for the needle 24. Starting from the initial configuration of Figure 3, under the pushing action of plunger 52, after removing the protective cap 25, it is possible to make the plug 7 slide to reduce the volume of the containment chamber 8 and eject a fraction (henceforth called "first fraction") of the injectable solution from the tubular body 3 through the outlet opening 22 of the dosing reservoir 20 until it reaches a pre-administering configuration represented in Figure 4 and 7.

In the pre-administering configuration:
- the plug 7 obstructs the inlet opening of the dosing reservoir 20;
- one fraction (henceforth called "second fraction") of the solution is contained inside the containment chamber 8; and
- the dosing reservoir 20 is filled with a fraction (henceforth called "third fraction") of injectable solution which represents of dose of solution to be administered to the patient.

Preferably the aforesaid third fraction is less than about 1/25 of the injectable solution and preferably equal to about 1/50 of the injectable solution. Preferably, the aforesaid dosing reservoir 20 has a volume of one millilitre or one tenth of a millilitre. The plug 7 includes a protruding appendix 16 suitable to pass through the inlet opening 21 of the dosing reservoir 20 and enter inside the latter in order to eject the third injectable solution from the dosing reservoir 20. This occurs for and during the administration of the third fraction of solution.

Preferably, the protruding appendix 16 has a cross section equal to the inlet opening of the dosing reservoir 20 or slightly smaller so that it can enter inside the dosing reservoir 20 sealingly engaging with the internal walls of the dosing reservoir 20 while sliding inside the latter.

As shown in Figure 3 and 9, the injection device 100 includes a vent opening 23, or vent way, suitable to be passed through by the second fraction of solution for ejecting the second fraction and to allow the protruding appendix 16 to pass through the inlet opening 21 of the dosing reservoir 20. Without the vent outlet 23, the second fraction of solution would make opposition to the plug 7 advancement to pass from the operative configuration of Figure 4 or 7 to the operative configuration of Figure 8, called "end-administration configuration".

Preferably, the vent opening 23 is provided with a vent channel 26.

Preferably, the vent opening 23 and the vent channel 26 are provided in the closing element 2. Preferably, the vent channel 26 is provided in parallel with the dosing reservoir 20.

Preferably, the vent opening 23 is initially closed, for instance by a removable plug 28.

The vent opening 23 is configured in such a way as to be opened starting from configuration of Figure 4, that is say from the pre-administering configuration.

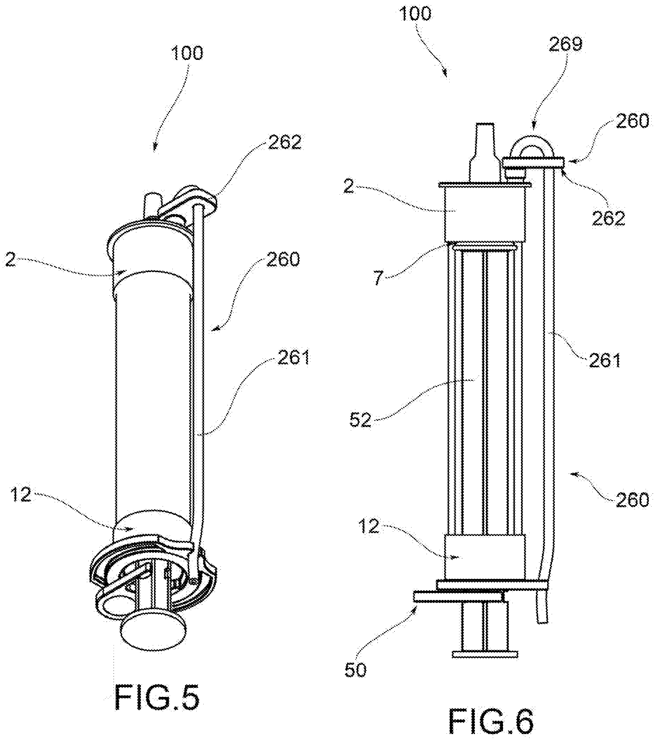

As shown in Figure 5, the injection device 100 includes one accessory 260 including a storing cannula 261 to collect the second fraction of solution.

From the above disclosure it is clear that establishing the quantity of the solution to administer to the patient occurs:
- ejecting one first fraction of the solution out of the tubular body 3;
- isolating one second fraction of the solution in the tubular body 3;
- isolating one third fraction of the solution inside the dosing reservoir 20.

In particular, the second fraction of solution is intended to be ejected from the tubular body 3 through the vent way 23 and be housed in the storing cannula 261 alongside the administration of the third fraction contained in the dosing reservoir 20.

The storing cannula 261 is provided with a first end 266 and a second end 267.

One passage is defined between the first 266 and the second 267 ends for the second fraction of solution. One storing reservoir 261' is defined between the first 266 and the second 267 ends for the second fraction of solution.

Preferably, the second end 267 of the storing cannula 261 is open to enable the emission of the air existing in the storing reservoir 261', such air would otherwise prevent the second fraction of solution from entering.

The cross-section of the storing cannula 261 is such to retain the second fraction of solution by capillarity inside the storing reservoir (261') even though the second end 267 of the storing cannula 261 is opened.

As shown in Figures 4 and 10, the storing cannula 261 is engageable with the vent opening 23, and in particular the first end 266 is engageable with the vent channel 26 of the injection device 100.

Starting from the pre-administering configuration of Figure 4, the vent opening 23 is opened by removing the plug 28. The storing cannula 261, and in particular the first end 266, is engaged with the vent opening 23, for example it is inserted in the vent channel 26 of the injection device 100.

The storing cannula 261 is long enough to house all the second fraction of solution.

Preferably, the second end 267 of the storing cannula 261 is housed into a housing 268 provided at the grasping portion 12.

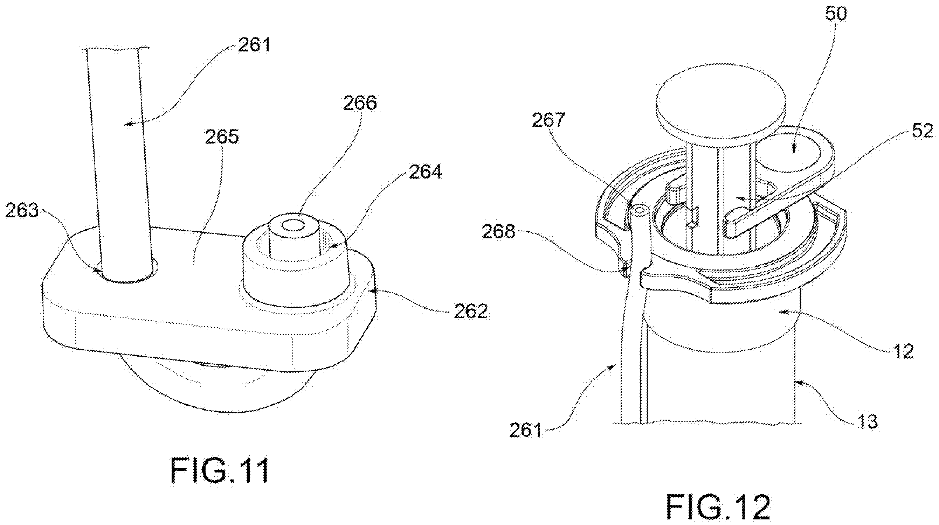

As shown in Figures 10 and 11, the accessory 260 also includes one support 262 to guarantee to position the storing cannula 261 in the vent channel 26 of the injection device 100.

The support 262 is for example a plate provided with a first seat 263 for housing the storing cannula 261 and a second seat 264 for housing a vent channel 26.

The first 263 and the second 264 seats are for example holes, or grooves provided in the body 265 of the support 262.

As shown in Figure 10, the vent channel 26 is housed in the second seat 264 provided in the support 262, and alongside in the vent channel 26 the first end 266 of the storing cannula 261 is housed. Such a construction choice improves the fixing of the storing cannula 261 to the injection device 100.

As shown in Figure 6, the storing cannula 261 runs in parallel with the containment body 3 of the injection device 100 between the closing element 2 and the grasping portion 12. Such a construction choice makes the injection device 100 with storing cannula 261 particularly compact and convenient during the administration of the injectable solution S, as the storing cannula 261 does not hinder the physician's actions.

In order to run in parallel to the containment body 3, the storing cannula 261 is provided with a curved portion 269.

Preferably, the injection device 100 further includes a stopper 50 to the sliding of the plunger 52.

As shown in Figure 12, the stopper 50 is applicable to the plunger 52,for example by interlocking and is preferably removable, and it is suitable to abut against the grasping portion 12.

The stopper 50 is suitable to abut against the grasping portion 12 at the configuration of Figure 4, that is at the pre-administering configuration. Advantageously, the stopper 50 prevents the plunger 52 from further advancing during the assembling step of the storing cannula 261. Advantageously, the stopper 50 prevents the plunger 52 from further advancing at a precise dose of solution S, for example at a dose equal to 0.1 ml of solution.

In the embodiment variant shown in the Figures 13 to 16, the injection device 100 is of the double-chamber type and includes:
- one first containment chamber 8 suitable to contain a first solid or liquid substance P;
- a first 6 and a second 7 plug, arranged inside the tubular body 3, so as to delimit the second containment chamber 9 therebetween the tubular body (3) and suitable to slide inside the tubular body 3 (for example as a result of a pushing or pulling force);
- a second containment chamber 9 suitable to contain a second liquid substance L, intended to be mixed inside the tubular body 3 with the first substance P for the reconstitution of an injectable solution.

In the embodiment variant wherein the injection device 100 is of the pre-filled type, the first chamber 8 contains a first substance P and the second chamber 9 contains a second substance L.

Preferably, the first substance P is a highly active substance, for instance a powder, a substance in granules or a sterile tablet or compacted powder. In case the first substance P is solid, it may be a crystallized or freeze-dried substance.

Preferably, the aforesaid second liquid substance L is a solvent for injectable use.

According to one embodiment (not shown in the Figures), the first plug 6, also called intermediate plug 6, includes at least a bypass channel which is originally in a closing condition and which is suitable to be taken to an opening condition after an external force occurrence, e.g. a pressure force acting on the plug. In the embodiment of Figure 13, the containment body 3 includes one inner wall provided with a recess 10 suitable to define a bypass channel, arranged downstream from the first plug 6.

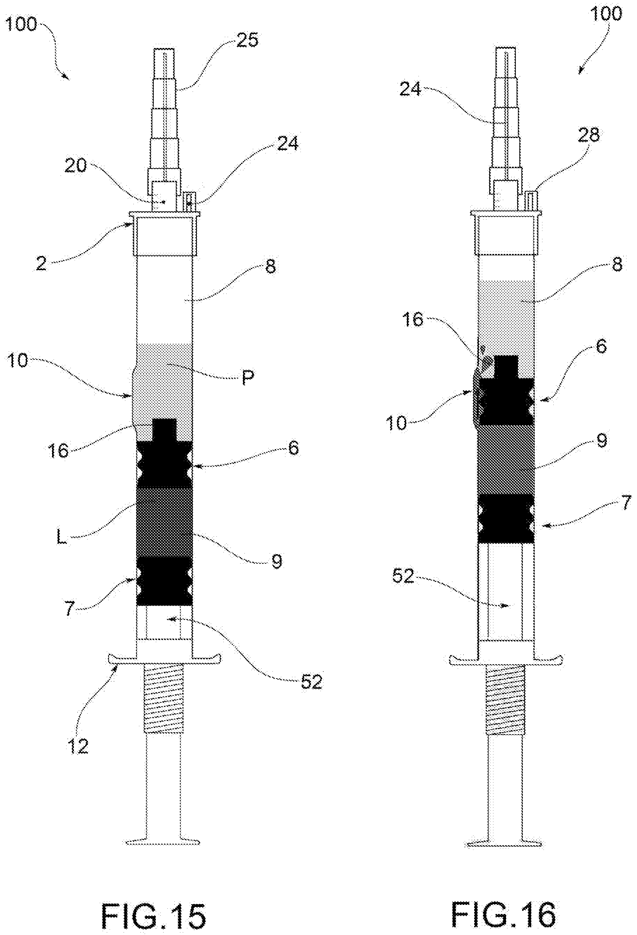

Starting from the initial configuration of Figure 15, following a pushing action of the plunger 52, the first 6 and the second 7 plugs are suitable to slide in the tubular body 3 until they reach a so called reconstitution configuration, represented in Figure 16, wherein two containment chambers 8 and 9 are in communication with each other through bypass 10 and the second substance 9 enters into the first chamber 8 to mix with the first substance.

Starting from the operative configuration of Figure 16, by further pushing the plunger 52, it is possible to further advance both plugs 6 and 7 until said plugs contact each other, eject the air existing in the first containment chamber 8 until they reach the operative configuration of Figure 3, also called "air-ejection end" configuration.

This invention also relates to an accessory 260 for an injection device 100, such accessory including at least one storing cannula 261 to collect one fraction of injectable solution S. Preferably, the accessory 260 also includes one support 262 suitable for guaranteeing the correct positioning of the storing cannula 261 on the injection device 100.

Innovatively, an injection device according to this invention avoids the dispersion of the ejected fraction during the injection step of the fraction of administration.

Advantageously, the storing cannula for containing the ejected fraction is easily applicable to the injection device with no need for substantial changes. Advantageously, an injection device according to this invention enables the physician not to contact the pharmaceutical substance.

Advantageously, an injection device according to this invention allows to administer very precise and reduced doses of an injection solution, e.g. of about one millilitre or one tenth of millilitre.

Subject to the principle of the invention, its embodiments and implementation details shall widely vary with respect to what has been disclosed and illustrated for exemplary non-limiting purposes, without departing from the scope of protection as defined in the enclosed claims.

## Claims

1. An injection device (100) for an injectable solution (S), including:
- a containment tubular body (3), closed at the front by a closing element (2) and at the back by a grasping portion (12), inside which at least one containment chamber (8, 9) is defined;
- a dosing reservoir (20) suitable to contain a fraction of the injectable solution (S), and having an inlet opening (21)in communication with the containment chamber (8) and an outlet opening (22);
- at least one plug (6, 7) arranged inside the tubular body (3) in order to delimit the containment chamber (8,9), said plug being suitable to slide inside the tubular body (3) and including a projecting appendix (16) suitable to pass through the inlet opening (21) of the dosing reservoir (20) and to enter inside the latter in order to eject said fraction of solution from the dosing reservoir (20);
- a vent outlet (23), provided with a vent channel (26), suitable to be passed through by a second fraction of solution to eject the second fraction and to allow the projecting appendix (16) to pass through the inlet opening (21) of the dosing reservoir (20);
**characterized by** including an accessory (260) having a storing cannula (261) being engageable with the vent opening (23), and provided with a first end (266) and a second end (267) between which a storing reservoir (261') for the second fraction of solution is defined, wherein the cross-section of the storing cannula (261) is such to retain the second fraction of solution by capillarity inside the storing reservoir (261').

2. The injection device (100) according to claim 1, wherein the storing cannula (261) is long enough to house all the second fraction of solution.

3. The injection device (100) according to claim 1 or 2, wherein the second end (267) of the storing cannula (261) is open in order to allow the air existing in the storing reservoir (261') to exit.

4. The injection device (100) according to any preceding claim, wherein the first end (266) of the storing cannula (261) is inserted or insertable in the vent channel (26) of the injection device (100).

5. The injection device (100) according to any preceding claim, wherein the second end (267) of the storing cannula (261) is inserted or insertable in a housing (268) being provided for at the grasping portion (12).

6. The injection device (100) according to any preceding claim, wherein the storing cannula (261) runs in parallel to the containment body (3) between the closing element (2) and the grasping portion (12).

7. The injection device (100) according to any preceding claim, wherein the accessory (260) also includes a support (262) to position the storing cannula (261) on the injection device (100), and wherein the support (262) is provided with a first seat (263) to house the storing cannula (261) and a second seat (264) to house the vent channel (26).

8. The injection device (100) according to any preceding claim, including a plunger (52) suitable to push the plug (6, 7), and a stopper (50) for the sliding of the plunger (52), said stopper being applicable by interlocking, for example, to the plunger (52) and it is suitable to abut against the grasping portion (12) in a pre-administering configuration.

9. The injection device (100) according to any preceding claim, wherein said fraction is less than about 1/25 of the injectable solution (S), preferably equal to about 1/50 of the injectable solution (S).

10. The injection device (100) according to any preceding claim, of the type being prefilled and with single chamber, wherein the containment chamber (8) contains an injectable solution (S).

11. The injection device (100) according to any claim 1 to 9, including:
- a first (8) and second (9) containment chambers being hermetically separated from one another;
- a first solid or liquid substance (P), contained in the first containment chamber (8);
- a first (6) and a second (7) plugs, arranged inside the tubular body (3), so as to define the second containment chamber (9) therebetween the tubular body (3) and adapted to slide inside the tubular body (3);
- a second liquid substance (L), contained inside the second containment chamber (9), intended to be mixed inside the tubular body (3) with the first substance to reconstitute an injectable solution;
- a bypass channel (10) between the first (8) and second (9) containment chamber suitable to allow mixing the first substance (P) and the second substance (L) in order to reconstitute the injectable solution (S).

## Patentansprüche

1. Eine Injektionsvorrichtung (100) für eine Injektionslösung (S), die folgende Merkmale aufweist:
- einen Behältnisrohrkörper (3), der an der Vorderseite durch ein Verschlusselement (2) und an der Rückseite durch einen Griffabschnitt (12) verschlossen ist, in dem zumindest eine Behältniskammer (8, 9) definiert ist;
- ein Dosierreservoir (20), das geeignet ist, um einen Bruchteil der Injektionslösung (S) zu beinhalten, und eine Einlassöffnung (21), die in Verbindung mit der Behältniskammer (8) steht, und eine Auslassöffnung (22) aufweist;
- zumindest einen Stöpsel (6, 7), der im Inneren des Rohrkörpers (3) angeordnet ist, um die Behältniskammer (8, 9) abzugrenzen, wobei der Stöpsel geeignet ist, um im Inneren des Rohrkörpers (3) zu gleiten, und einen vorstehenden Anhang (16) aufweist, der geeignet ist, um durch die Einlassöffnung (21) des Dosierreservoirs (20) zu verlaufen und ins Innere von letzterem zu gelangen, um den Bruchteil der Lösung aus dem Dosierreservoir (20) auszustoßen;
- einen Entlüftungsauslass (23), der mit einem Entlüftungskanal (26) versehen ist, der geeignet ist, um durch einen zweiten Bruchteil der Lösung zu laufen, um den zweiten Bruchteil auszustoßen und zu ermöglichen, dass der vorstehende Anhang (16) durch die Einlassöffnung (21) des Dosierreservoirs (20) verläuft;
**dadurch gekennzeichnet, dass** dieselbe einen Zusatz (260) mit einer Speicherkanüle (261) aufweist, die mit der Entlüftungsöffnung (23) in Eingriff bringbar ist, und mit einem ersten Ende (266) und einem zweiten Ende (267) versehen ist, zwischen denen eine Speicherreservoir (261') für den zweiten Bruchteil der Lösung definiert ist,
wobei der Querschnitt der Speicherkanüle (261) derart ist, dass der zweite Bruchteil der Lösung durch Kapillarwirkung im Inneren des Speicherreservoirs (261') gehalten wird.

2. Die Injektionsvorrichtung (100) gemäß Anspruch 1, bei der die Speicherkanüle (261) lang genug ist, um den gesamten zweiten Bruchteil der Lösung unterzubringen.

3. Die Injektionsvorrichtung (100) gemäß Anspruch 1 oder 2, bei der das zweite Ende (267) der Speicherkanüle (261) offen ist, um zu ermöglichen, dass Luft, die in dem Speicherreservoir (261') vorhanden ist, austreten kann.

4. Die Injektionsvorrichtung (100) gemäß einem der vorherigen Ansprüche, bei der das erste Ende (266) der Speicherkanüle (261) in den Entlüftungskanal (26) der Injektionsvorrichtung (100) eingeführt ist oder einführbar ist.

5. Die Injektionsvorrichtung (100) gemäß einem der vorherigen Ansprüche, bei der das zweite Ende (267) der Speicherkanüle (261) in ein Gehäuse (268), das an dem Griffabschnitt (12) vorgesehen ist, eingeführt ist oder einführbar ist.

6. Die Injektionsvorrichtung (100) gemäß einem der vorherigen Ansprüche, bei der die Speicherkanüle (261) zwischen dem Verschlusselement (2) und dem Griffabschnitt (12) parallel zu dem Behältniskörper (3) verläuft.

7. Die Injektionsvorrichtung (100) gemäß einem der vorherigen Ansprüche, bei der der Zusatz (260) außerdem einen Träger (262) aufweist, um die Speicherkanüle (261) an der Injektionsvorrichtung (100) zu positionieren, und wobei der Träger (262) mit einer ersten Auflage (263) zum Unterbringen der Speicherkanüle (261) und mit einer zweiten Auflage (264) zum Unterbringen des Entlüftungskanals (26) versehen ist.

8. Die Injektionsvorrichtung (100) gemäß einem der vorherigen Ansprüche, die einen Kolben (52), der geeignet ist, um auf den Stöpsel (6, 7) zu drücken, und ein Anschlagselement (50) für das Gleiten des Kolbens (52) aufweist, wobei das Anschlagselement durch Verriegeln beispielsweise an den Kolben (52) anwendbar ist und geeignet ist, um in einer Vorverabreichungsausbildung an den Griffabschnitt (12) anzugrenzen.

9. Die Injektionsvorrichtung (100) gemäß einem der vorherigen Ansprüche, bei der der Bruchteil weniger als etwa 1/25 der Injektionslösung (S) beträgt, vorzugsweise etwa gleich 1/50 der Injektionslösung (S).

10. Die Injektionsvorrichtung (100) gemäß einem der vorherigen Ansprüche, die von dem Typ ist, der vorgefüllt ist und eine einzelne Kammer aufweist, wobei die Behältniskammer (8) eine Injektionslösung (S) beinhaltet.

11. Die Injektionsvorrichtung (100) gemäß einem der Ansprüche 1 bis 9, die folgende Merkmale aufweist:
- eine erste (8) und eine zweite (9) Behältniskammer, die hermetisch voneinander getrennt sind;
- eine erste feste oder flüssige Substanz (P), die in der ersten Behältniskammer (8) beinhaltet ist;
- einen ersten (6) und einen zweiten (7) Stöpsel, die im Inneren des Rohrkörpers (3) angeordnet sind, um so die zweite Behältniskammer (9) dazwischen in dem Rohrkörper (3) zu definieren, und angepasst sind, um im Inneren des Rohrkörpers (3) zu gleiten;
- eine zweite flüssige Substanz (L), die im Inneren der zweiten Behältniskammer (9) beinhaltet ist, die dafür gedacht ist, im Inneren des Rohrkörpers (3) mit der ersten Substanz gemischt zu werden, um eine Injektionslösung neu zusammenzustellen;
- einen Umleitungskanal (10) zwischen der ersten (8) und der zweiten (9) Behältniskammer, der geeignet ist, um ein Mischen der ersten Substanz (P) und der zweiten Substanz (L) zu ermöglichen, um die Injektionslösung (S) neu zusammenzustellen.

## Revendications

1. Dispositif d'injection (100) pour une solution injectable (S), comportant :
- un corps tubulaire de confinement (3), fermé à l'avant par un élément de fermeture (2) et à l'arrière par une portion de préhension (12), à l'intérieur duquel au moins une chambre de confinement (8, 9) est définie ;
- un réservoir de dosage (20) approprié pour contenir une fraction de la solution injectable (S), et ayant une ouverture d'entrée (21) en communication avec la chambre de confinement (8) et une ouverture de sortie (22) ;
- au moins un obturateur (6, 7) agencé à l'intérieur du corps tubulaire (3) afin de délimiter la chambre de confinement (8, 9), ledit obturateur étant approprié pour coulisser à l'intérieur du corps tubulaire (3) et comportant un appendice en saillie (16) approprié pour passer à travers l'ouverture d'entrée (21) du réservoir de dosage (20) et pour entrer à l'intérieur de ce dernier afin d'éjecter ladite fraction de solution du réservoir de dosage (20) ;
- une sortie d'évent (23), munie d'un canal d'évent (26), appropriée pour être traversée par une seconde fraction de solution pour éjecter la seconde fraction et pour permettre à l'appendice en saillie (16) de passer à travers l'ouverture d'entrée (21) du réservoir de dosage (20) ;
**caractérisé en ce qu'**il comprend un accessoire (260) ayant une canule de stockage (261) qui peut s'engager avec l'ouverture d'évent (23), et munie d'une première extrémité (266) et d'une seconde extrémité (267) entre lesquelles un réservoir de stockage (261') pour la seconde fraction de solution est défini,
dans lequel la section transversale de la canule de stockage (261) est telle qu'elle retient la seconde fraction de solution par capillarité à l'intérieur du réservoir de stockage (261').

2. Dispositif d'injection (100) selon la revendication 1, dans lequel la canule de stockage (261) est suffisamment longue pour loger l'ensemble de la seconde fraction de solution.

3. Dispositif d'injection (100) selon la revendication 1 ou 2, dans lequel la seconde extrémité (267) de la canule de stockage (261) est ouverte afin de permettre à l'air existant dans le réservoir de stockage (261') de sortir.

4. Dispositif d'injection (100) selon une quelconque revendication précédente, dans lequel la première extrémité (266) de la canule de stockage (261) est insérée ou peut être insérée dans le canal d'évent (26) du dispositif d'injection (100).

5. Dispositif d'injection (100) selon une quelconque revendication précédente, dans lequel la seconde extrémité (267) de la canule de stockage (261) est insérée ou peut être insérée dans un logement (268) qui est ménagé à cette fin au niveau de la portion de préhension (12).

6. Dispositif d'injection (100) selon une quelconque revendication précédente, dans lequel la canule de stockage (261) chemine parallèlement au corps de confinement (3) entre l'élément de fermeture (2) et la portion de préhension (12).

7. Dispositif d'injection (100) selon une quelconque revendication précédente, dans lequel l'accessoire (260) comporte également un support (262) pour positionner la canule de stockage (261) sur le dispositif d'injection (100), et dans lequel le support (262) est muni d'un premier siège (263) pour loger la canule de stockage (261) et d'un second siège (264) pour loger le canal d'évent (26).

8. Dispositif d'injection (100) selon une quelconque revendication précédente, comportant un piston (52) approprié pour pousser l'obturateur (6, 7), et un bouchon (50) pour le coulissement du piston (52), ledit bouchon étant applicable par verrouillage mutuel, par exemple, au piston (52) et il est approprié pour buter contre la portion de préhension (12) dans une configuration de pré-administration.

9. Dispositif d'injection (100) selon une quelconque revendication précédente, dans lequel ladite fraction est inférieure à environ 1/25 de la solution injectable (S), de préférence égale à environ 1/50 de la solution injectable (S).

10. Dispositif d'injection (100) selon une quelconque revendication précédente, du type qui est pré-rempli et avec une seule chambre, dans lequel la chambre de confinement (8) contient une solution injectable (S).

11. Dispositif d'injection (100) selon l'une quelconque des revendications 1 à 9, comportant :
- des première (8) et seconde (9) chambres de confinement qui sont hermétiquement séparées l'une de l'autre ;
- une première substance solide ou liquide (P), contenue dans la première chambre de confinement (8) ;
- des premier (6) et second (7) obturateurs, agencés à l'intérieur du corps tubulaire (3), de façon à définir la seconde chambre de confinement (9) entre eux le corps tubulaire (3) et adaptés pour coulisser à l'intérieur du corps tubulaire (3) ;
- une seconde substance liquide (L), contenue à l'intérieur de la seconde chambre de confinement (9), destinée à être mélangée à l'intérieur du corps tubulaire (3) avec la première substance pour reconstituer une solution injectable ;
- un canal de contournement (10) entre les première (8) et seconde (9) chambres de confinement approprié pour permettre le mélange de la première substance (P) et de la seconde substance (L) afin de reconstituer la solution injectable (S).
